# EUROPÄISCHE PATENTSCHRIFT

(11) **EP 3 768 331 B1**
(45) Veröffentlichungstag und Bekanntmachung des Hinweises auf die Patenterteilung: **17.07.2024**
(21) Anmeldenummer: 19714303.5
(22) Anmeldetag: 13.03.2019
(51) Int. Cl.: A61L 2/10, A61L 2/22

(54) **VORRICHTUNG UND VERWENDUNG EINER SOLCHER VORRICHTUNG ZUR DESINFEKTION VON ROHRLEITUNGEN**
DEVICE AND ITS USE FOR DISINFECTING PIPELINES
DISPOSITIF ET SON USAGE POUR LA DÉSINFECTION DE TUYAUTERIES

(30) Priorität: 22.03.2018 AT 502402018; 13.12.2018 AT 511162018
(43) Veröffentlichungstag der Anmeldung: 27.01.2021
(73) Patentinhaber: Lesstec AG, 8702 Zollikon (CH)
(72) Erfinder: Mock, Manfred, 8225 Pöllau (AT)
(74) Vertreter: ETL IP Patent- und Rechtsanwaltsgesellschaft mbH
(86) Internationale Anmeldenummer: PCT/AT2019/060082
(87) Internationale Veröffentlichungsnummer: WO 2019/178624

(56) Entgegenhaltungen:
- WO-A1-2011/153288
- CN-A- 104 740 670
- US-A- 5 072 487

## Beschreibung

Die Erfindung betrifft eine Vorrichtung zur Desinfektion von Innenwänden von Rohrleitungen, entsprechend dem Oberbegriff des Anspruches 1. Die im Folgenden offenbarte Desinfektion von Behältern oder Bauwerken dient lediglich der Darstellung der Erfindung, ist jedoch nicht Teil der beanspruchten Erfindung.

In (trink)wasserdurchflossenen Rohren, Behältern oder Bauwerken, gleich welchen Querschnittes, kommt es mit der Zeit zur Bildung von mehr oder weniger starken Biofilmen, die sich an der Innenrohrwand bilden und als Lebensgrundlage für Mikroorganismen dienen. Solange diese Biofilme frei von schädlichen Krankheitskeimen und Erregern sind, ist dies nicht weiter problematisch.

In diesen Biofilmen können sich jedoch auch pathogene Keime (Bakterien und/oder Viren wie z.B. Escherichia coli, Enterokokken, Legionellen oder Pseudomonas aeruginosa), aber auch Pilze und deren Sporen, bei Lichteinfall auch Algen, ansiedeln, die sich dort vermehren und mit dem durch das Rohr durchfließenden Wasser ausgespült werden. Diese Mikroorganismen können dann zu den einzelnen Verbrauchern (z.B. bei Trinkwasserversorgungssystemen zu den einzelnen Haushalten) gelangen und zu einer Gesundheitsgefährdung für Menschen und Tieren führen.

Deshalb ist es notwendig, Trinkwasserversorgungssysteme bei Bedarf zu reinigen, zu beproben und gegebenenfalls auch zu desinfizieren.

### Desinfektion:

Beim Desinfizieren versetzt man ein System in einen Zustand (Milieu), in dem Mikroorganismen abgetötet oder derart geschädigt werden, dass ein Wachstum oder eine Vermehrung nicht mehr zu erwarten ist. Weiters bedeutet Desinfektion auch bei Vorhandensein von Viren deren Inaktivierung. Dieser Zustand ist im Allgemeinen zeitlich begrenzt und muss gegebenenfalls immer wieder wiederholt werden.

Die derzeit gängigen Verfahren der Desinfektion von (trink)wasserdurchflossenen Rohren, Behältern oder Bauwerken nach dem Stand der Technik beruhen auf dem Einsatz von wässerigen Desinfektionslösungen oder Gasen.

Dabei werden im Durchflussverfahren oder Standverfahren wässerige Desinfektionslösungen in die zu desinfizierenden Bauteile zu- bzw. ein bzw. durchgeleitet.

Für Trinkwasserversorgungssysteme sind Maßnahmen nach den Vorgaben der ÖVWG RL W 55 oder DVGW W 291 anzuwenden, unter Einsatz der o.a. Desinfektionsmittel. Folgende Desinfektionstechniken werden derzeit eingesetzt (ohne Erläuterung der Vorreinigung und der Nachspülung) :
- Im Durchflussverfahren wird dem in einer Leitung fließenden Wasser ein Desinfektionsmittel i.R. meist Chlor oder Chlorlösung je nach Erfordernis in unterschiedlichen Konzentrationen und unterschiedlichen Zeiten zugesetzt.
- Beim Standverfahren lässt man die zur Desinfektion verwendete Desinfektionslösung (z.B. stärker gechlortes Wasser) für einen längeren Zeitraum (etwa 24 - 48 Stunden) in den Rohrleitungen stehen und leitet sie anschließend ab.

Der Nachteil der o.a. Techniken ist der, dass einerseits zu entsorgende Desinfektionslösungen in größeren Mengen anfallen und andererseits das z.B. gechlorte Wasser anschließend langwierig aus den Leitungen und Behältern entfernt werden muss. US5072487A offenbart eine Vorrichtung zur Desinfektion des Inneren von Rohren, die sowohl Düsen als auch UV-Lampen umfasst.

### Problemstellung:

Problematisch wird es trotz der erläuterten Desinfektion aber immer dann, wenn sich im Biofilm sehr resistente Keime, wie z.B. Pseudomonas aeruginosa, einnisten. Hat sich dieser Keim im Biofilm einmal manifestiert, ist mit einer chemischen Desinfektion - wie o.a.- keine Möglichkeit gegeben, diesen nachhaltig aus dem Rohr bzw. Biofilm zu entfernen. Bei Chloreinsatz schützt sich dieser nämlich mittels einer wachsähnlichen Schicht und "sitzt" sozusagen die Gefahrensituation aus.

Es ist Ziel und Aufgabe der Erfindung, eine Vorrichtung anzugeben, die in der Lage ist, auch mit derart resistenten Keimen fertig zu werden.

Erfindungsgemäß werden diese Ziele durch eine Vorrichtung erreicht, die die im kennzeichnenden Teil des Anspruches 1 angegebenen Merkmale aufweist. Mit anderen Worten, es wird durch die entleerte und gegebenenfalls vorgereinigte Rohrleitung, eine Sprühkette bewegt, die zumindest eine Düse zur Abgabe und Vernebelung des Desinfektionsmittels und/oder zumindest eine Quelle für UV Licht, insbesondere eine UVC-Quelle aufweist. Die Sprühkette ("Sprüh" wegen des Versprühens von Nebel und/oder elektromagnetischen Strahlen, letztere je brauchbarer, je "härter") ist, wie im Folgenden näher erläutert, so aufgebaut, dass die Düse und/oder Quelle Abstand vom Boden und den seitlichen Wänden des Rohres, aufweist.

Die erfindungsgemäße Vorrichtung weist eine Sprühkette auf, die in die vorgereinigte oder ungereinigte Rohrleitung, eingeführt und dort aktiviert wird, somit das Desinfektionsmittel in vernebelter Form abgibt und von einer sogenannten Betriebstrommel aus, gesteuert wird. Die Sprühkette kann von einem vorher in die Rohrleitung, eingeführten Zugmittel (Kette, Seil, etc.) gezogen werden, sie kann an ein (ferngelenktes) Fahrzeug angehängt werden, oder auf andere Weise durch den zu reinigenden Bereich bewegt werden.

Mit Hilfe der Sprüh und Nebeldüsen kann erfindungsgemäß auch Alkohol in das Gebäude eingebracht werden, welcher den kompletten Biofilm vernichtet und die pathogenen Keime an ihrer Vermehrung und Ausbreitung hindert. Das Alkohol-Sprühen mit hohem Druck bringt auch eine mechanische Ablösung des Biofilms mit sich. Ein mechanisches Abscheren ist sozusagen mit inbegriffen.

Das Vorsehen entsprechender Lichtquellen, ihre Energiezufuhr über Stromleitungen und die Aktivierung mittels Schaltern ist auch ohne weitere Erläuterung direkt verständlich, dass zumeist mehrere derartige Quellen vorgesehen sind, die in unterschiedliche Richtungen gerichtet sind, ist in Kenntnis der Erfindung wohl naheliegend.

Eine UV Behandlung an sich ist bei der Desinfektion zur Trinkwasseraufbereitung als Stand der Technik zu bezeichnen. Doch wird dabei Trinkwasser im Durchlaufverfahren durch sogenannte UVC Desinfektionsanlagen geleitet und anschließend in das Trinkwassernetz eingespeist. Diese Anlagen können auch direkt in die Wasserleitungen selbst eingebaut werden oder es wird die UV-Anlage bei einer Wasserversorgung unmittelbar nach der Wassergewinnung und einer eventuellen Aufbereitung, auf jeden Fall aber vor dem Verteilernetz und eventuellen Speicherbehältern, eingebaut.

Beim Stand der Technik kann mit dem Befüllen des Rohres mit sauberem Wasser nach der Reinigung und dem üblichen Nachspülen mit reinem Wasser, das aber noch nicht zur weiteren Verwendung gebracht, sondern abgelassen wird, kann sich der natürliche Biofilm mit seiner "normalen" Besiedelung im Laufe der Zeit wieder manifestieren.

Durch den Einsatz der erfindungsgemäßen Technologie kann man alle oben angeführten Nachteile vermeiden und selbst Pseudomonas aeruginosa wirkungsvoll bekämpfen und so eine den gesetzlichen Anforderungen entsprechende, lang anhaltende Keimfreiheit in brauchwasserdurchflossenen wie auch lang anhaltende Trinkwassertauglichkeit in trinkwasserdurchflossenen Rohren, Behältern oder Bauwerken erzielen.

Es können erfindungsgemäß alle bekannten Desinfektionsmittel, flüssig oder gasförmig, verwendet werden, Beispiele sind:
Halogenhältige Chemikalien, wie Chlor, Chlorbleichlauge (Natriumhypochlorit-Lösung), Chlorkalk (Calciumhypochlorit), Chlordioxid; Sauerstoffhältige Chemikalien, wie Wasserstoffperoxid, Kaliumpermanganat, Ozon, Peressigsäure; Stickstoffhältige Chemikalien, wie quarternäre Ammoniumverbindungen, Benzalkoniumchlorid, Cetylalkoniumchlorid; Alkohole, wie Isopropanol.

Insbesondere Licht im Wellenlängenbereich zwischen 200 und 300 Nanometern (nm) mit einem ausgeprägten Maximum bei ca. 265 nm eignet sich für die Desinfektion von Trinkwasser. Die optimale Wellenlänge kann je nach Mikroorganismus leicht variieren und gegebenenfalls berücksichtigt werden. Ein Vorteil einer solchen UV-Desinfektion gegenüber herkömmlichen Verfahren (chemische Desinfektion) ist der Umstand, dass es Bakterien und Viren nicht möglich ist, Resistenzen gegen ultraviolette Strahlung aufzubauen; in Kombination ergibt sich eine unerwartete Verbesserung gegenüber der Einzelwirkung.

Es ist bereits gelungen, Wasser mithilfe von ultravioletten Leuchtdioden (UV-LEDs) zu desinfizieren. Die Bestrahlung mit UV-Licht zerstört das Erbgut von Bakterien, Viren und Sporen (allgemein: Keimen) und verhindert dadurch die Vermehrung der Organismen. In Sonderfällen können vorteilhafterweise auch noch kurzwelligere Strahlen eingesetzt werden, auch wenn deren Abgabe besonderen Sicherheitsmaßnahmen unterliegen muss.

Es konnte festgestellt werden, dass es auch bei hartnäckigen Keimen aller Art, die an Wänden von wasserführenden Gebilden nisten, durch das erfindungsgemäße Verfahren, insbesondere in der Kombinationsvariante, problemlos möglich ist, die der Gesetzeslage entsprechende Hygiene einzuhalten.

Die Erfindung wird im Folgenden anhand der Zeichnung näher beschrieben, dabei zeigt, rein schematisch:

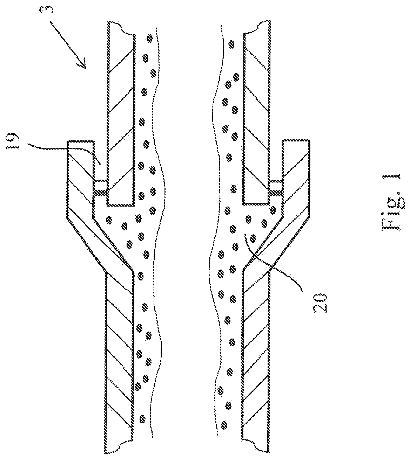

die Fig. 1 einen Rohrzusammenschluss mit Verunreinigungen,
die Fig. 2 eine sogenannte Betriebstrommel,
die Fig. 3 eine erfindungsgemäß einsetzbare Sprühkette in Seitenansicht und
die Fig. 4 in axialer Ansicht

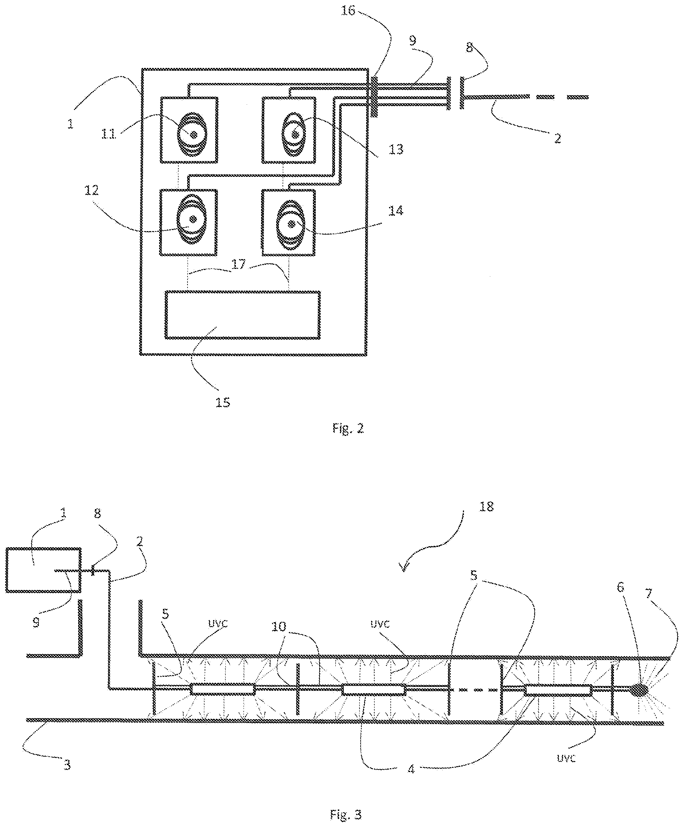

Bevorzugt wird bei der Anwendung des Verfahrens mittels einer sogenannten Betriebstrommel, wie sie in Fig. 2 schematisch abgebildet ist, eine Sprühkette mit zumindest einem, bevorzugt mehreren Sprühköpfen zur Abgabe des Desinfektionsmittels (kurz "Mittel") ausgerüstet. Durch die Abgabe des Mittels in Form eines feinen Sprühnebels erhöht sich dessen Wirksamkeit, vermutlich durch die Vergrößerung der Oberfläche, deren starke Krümmung, und den so bewirkten intensiven Kontakt mit dem Sauerstoff der Umgebungsluft.

Es können erfindungsgemäß UVC bzw. UV LED Lampen, wie sie in Fig. 3 schematisch abgebildet ist, in das möglichst entleerte und vorgereinigte Rohrleitungssystem eingeführt, sodann werden die Lampen aktiviert und das Rohr unter Einhaltung bestimmter Reaktionszeiten von innen bestrahlt. Die Kombination steigert die Wirksamkeit des Mittels, vermutlich durch den weiter erhöhten Energieeintrag, noch weiter.

Zur Betriebstrommel 1 ist auszuführen: Sie ist die zentrale Versorgungs- und Steuerungseinheit für die Sprühkette. Die eigentliche Steuerungseinheit 15 steuert die Ab- bzw. Aufrolleinheiten von 11, 12, 13 und 14 über die Steuerleitungen 17 und rollt die Verbindungsschläuche und -kabel je nach gebrauchter Länge ab bzw. auf. Die Verbindungsschläuche für Desinfektionsmittel, elektr. Strom, Datenkabel und Kamerakabel werden in einer Kupplung 8 zusammengefasst und über eine Auslassrolle 16 aus der Betriebstrommel 1 ausgeleitet. Über die Kupplung 8 wird die Sprühkette an die Verbindungsschläuche bzw. Verbindungskabel angekoppelt. Desinfektionsmittel, etc. und elektrischer Strom werden von extern in die entsprechenden Anschlussstellen bei den Ab- bzw. Aufrolleinheiten 11, 12, 13 und 14 eingespeist. In 12 integriert ist eine Pumpe, die das Desinfektionsmittel in den Verbindungsschlauch zu 12 hineinpumpt für die nachfolgende Zerstäubung über einen Sprühkopf 6.

Die Versorgungsleitungen und Versorgungskabel 9 sind mit einer jeweiligen Länge von bis zu 500 m auf den Ab- bzw. Aufrolleinheiten 11, 12, 13 und 14 aufgerollt und funktionieren analog einer elektrisch betriebenen Haspel. Die Versorgungsleitungen 9 sind nach der Kupplung 8 in zu einer einzigen - umwickelten - Versorgungsleitung 2 zusammengefasst. Die Betriebstrommel 1, als gesamte Einheit, kann auf einem eigenen Träger, Anhänger oder in einem Kfz montiert werden.

Das Einbringen und das Bewegen der Sprühkette kann auf verschiedene Weise erfolgen, es kann ein Zugmittel zuerst eingebracht werden und dann die Sprühkette gezogen werden, es kann unter Umständen die Sprühkette geschoben werden, oder an ihrem Kopf ist ein Fahrzeug angebracht, das die Sprühkette hinter sich herzieht.

Die Betriebstrommel dient als Steuerungsanlage zur Regelung der Sprühkette, der Vortriebsgeschwindigkeit im Rohr und somit der Verweilzeit der Bestrahlung und der allgemeinen Prozessüberwachung sowie Prozessprotokollierung.

In Rohrleitungsystemen 3 wird speziell in schwer zugänglichen Stellen ein Desinfektionsmittel in Richtung zu diesen Stellen gesprüht. Dieses wirkt besonders bakterizid und kann, in zerstäubter oder vernebelter Form, auch in entlegene Winkel, vordringen. Darüber hinaus ist der Sprühnebel in der Lage, in Biofilme 20 einzudringen und daher auch in der Tiefe zu wirken. Dieser Sprühvorgang kann unabhängig vom anderen Betrieb der Sprühkette, bevorzugt durch die Sprühdüsen, die an der Sprühkette vorgesehen sind, durchgeführt werden. Das Fluid mit dem Mittel kann über einen Schlauch zugeführt werden, der an der Sprühkette befestigt ist.

Sowohl die wässerigen Desinfektionsmittel als auch die gasförmigen werden bevorzugt mittels Druckleitungen, die mit der Sprühkette verbunden sind, in das Rohr eingeführt und mittels Pumpen und ferngesteuerten Ventilen passend versprüht. In Sonderfällen kann das Fluid aber auch aus einem mitbewegten Vorratstank gepumpt werden.

Zur Sprühkette 18 ist speziell auszuführen: Sie besteht im Wesentlichen aus der Versorgungsleitung 2, den Sprühköpfen 6, den Abstandhaltern 5 und Verbindungsadaptern 10 und(oder einer oder mehreren Strahlenquellen 4. Sie wird je nach benötigter Länge vormontiert, in das Rohr 3 voreingeschoben, anschließend an der Kupplung 8 angekuppelt und dann vollständig ins Rohr 3 eingebracht.

Die Sprühkette kann je nach Anwendungsfall entweder
- mechanisch eingeschoben
- mit dem Wasser im Rohr eingeschwemmt,
- mit Druckluft eingeblasen,
- mittels Seilzug oder Roboter ziehend geführt
ins Rohr 3 eingebracht werden.

Die Sprühkette wird also mit annähernd radial zu Ihrer "Wirbelsäule" orientierten Abstandhaltern 5, deren Länge variabel und abhängig vom Durchmesser des Rohres - allgemein des Fluidleiters - ist, den UVC Strahlenquellen 4, den Verbindungsadaptern 10 und Sprühköpfen 6 in das Rohr 3 eingeführt. Die Sprühkette wird mittels einer Kupplung 8 an die Versorgungsleitungen der Betriebstrommel 1 angeschlossen. Die Verbindungsadapter 10 haben einen integrierten Kabeldurchlass und sind für die Stabilität der Sprühkette in Längsrichtung günstig - die Abstandhalter 5 (zumindest drei jeweils an gleicher oder benachbarter axialer Stelle sind empfehlenswert) sorgen für die Stabilität der Sprühkette in Querrichtung.

Die Dauer des Einnebelns und/oder die Bestrahlungsdauer und Bestrahlungsintensität durch die Lichtlampen hängt von der Verschmutzung allgemein, von der Stärke/Dicke des Biofilms und des Rohrmaterials ab, und liegt nach internen Untersuchungen im Durchschnitt bei 0,5- 4 Sekunden bzw. 0,1-10 Watt/ cm2. Längere Bestrahlungszeiten und höhere Intensitäten sind natürlich auch möglich. Der Nebel kann am Ende der Behandlung oder auch während der Behandlung durch Ventilatoren an zumindest einer Seite des zu reinigenden Gebäudes abgezogen oder in Bewegung (hin-her) gehalten werden, einerseits um nach erfolgter Reinigung rasch in den Betriebszustand zurückzukommen, andererseits um die Wirkung durch die Bewegung zu erhöhen.

Über die Versorgungsleitung 2 werden der elektrische Strom für die Lampen 4, das Desinfektionsmittel (allgemein: das Mittel) für die Versprühung mittels der Sprühköpfe 6 in das Rohr eingeführt.

Die Betriebstrommel 1 stellt auch die Energiequelle für die Versorgung der Lampen 4 mit elektr. Strom dar. Über die Betriebstrommel 1 wird auch die Versorgung mit Desinfektionsmittel sichergestellt. Das Desinfektionsmittel und das Gas werden mittels Pumpen über die Versorgungsleitung 2 in das Rohr 3 eingeleitet. Die Desinfektionsmittelzerstäubung wird über die Sprühköpfe 6 durchgeführt, die gegebenenfalls einzeln mittels Magnetventilen aktiviert werden.

Die Grobreinigung - Vorreinigung der Rohre etc. kann entweder mittels Wasserspülungen oder durch Molchen erfolgen, und geg. nochmals eine Spülung oder Molchung. Im nächsten Schritt erfolgt dann die Desinfektion mittels UVC Licht und/oder dem Mittel. Das Mittel wird mittels eigener Druckleitung, die in der Versorgungsleitung 2 integriert ist, in das Rohr 3 hineingepumpt und dort lokal mittels der Sprühköpfe 6 versprüht. Am Ende erfolgt ein Ausspülen des Rohres mit Wasser.

Der Durchmesser der Rohre, in welche eine (jeweils bautechnisch angepasste) Sprühkette eingeführt werden kann, beträgt mindestens 1 Zoll (25,4 mm) und max. DN 1500 mm; bei nichtkreisförmigem Querschnitt gelten analoge Werte, die der Fachmann in Kenntnis der Erfindung leicht feststellen kann.

Die Sprühdüsen 6 sind bevorzugt nacheinander in unterschiedliche Umfangsrichtung orientiert, um bei etwa mittiger Position der Sprühköpfe im Bauteil/Rohr rasch und zuverlässig eine gleichmäßige Einnebelung des Inneren und damit der gesamten Oberfläche des Gebäudes (Rohres) zu erreichen.

Zwischen den einzelnen Strahlern 4 befinden sich - je nach Rohrdurchmesser - unterschiedlich große Abstandhalter 5, die gewährleisten, dass die Strahler annähernd (+/- 10 %) mittig im Rohr 3 positioniert sind und so die UVC Strahlen gleichmäßig im Rohr 3 abgegeben werden. Die Anzahl der UVC Strahlenquellen 4 ist je nach Anwendungsfall variierbar und hängt vom Grad der Verkeimung, der notwendigen Verweilzeit (Bestrahlungszeit) im Rohr 3, der vorgesehenen Arbeitsdauer und Ähnlichem ab. Bei starkem Abweichen vom Kreisquerschnitt in Gebäuden kann in Kenntnis der Geometrie ohne Probleme eine Anpassung erfolgen.

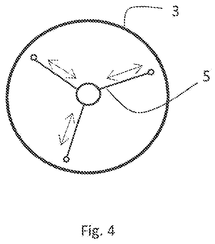

Die Sprühkette 18 weist, wie aus Fig. 4 gut ersichtlich, im Wesentlichen radial abstehende Abstandhalter 5 auf, die auch beim Verdrehen der Sprühkette um die Längsachse sicherstellen, dass sie stets im Abstand zum Boden und den Seitenwänden gehalten wird.

Die Abstandhalter 5 können, je nach Größe des Durchmessers des Rohres 3, mit unterschiedlichen Längen (beispielsweise längenverstellbar, in der Fig. 4 mit " <-----> " angedeutet, oder auswechselbar) montiert werden.

Die Abstandhalter 5 bestehen im Regelfall aus drei in Umfangsrichtung in unterschiedlichen Winkeln voneinander versetzten Armen (bevorzugt 120°), die bevorzugt am jeweiligen äußeren Ende jedes Arms Rollen (nur angedeutet, ohne Bezugszeichen) montiert haben, die ein leichteres Bewegen im Rohr 3 ermöglichen. Solche Arme werden je nach Bedarf in passendem axialen Abstand an der Kette der Sprühkette vorgesehen.

Es können die Abstandhalter auch nach Art einer Wendel axial und in Umfangsrichtung versetzt angeordnet sein, in Kenntnis der Erfindung und des jeweiligen Anwendungsgebietes ist es für den Fachmann ein Leichtes, hier seine Wahl zu treffen.

In der axialen Mitte der Abstandhalter 5 befinden sich vorne und hinten Verbindungskupplungen, sodass der Verbindungsadapter 10 hier angekoppelt werden kann. Verbindungsadapter 10 und Abstandhalter 5 haben jeweils mittig einen freien Querschnitt für den Durchgang der Versorgungsleitung 2.

An die Verbindungsadapter 10 (mit Dichtung) wird die UVC Lampe angekoppelt und dadurch wird die benötigte Steifheit erhalten, die notwendig ist, die Sprühkette 18 ins Rohr 3 einzuführen und zu bewegen. Neben dieser Steifigkeit einerseits weist die Sprühkette, insbesondere, wenn sie so ausgelegt ist, dass sie stets gezogen wird, eine Beweglichkeit in ihrer "Wirbelsäule" auf, die es ihr ermöglicht, eventuellen Krümmungen des Rohres oder Bauwerks zu folgen. Dies kann durch Kugelgelenke oder, einfacher, durch ausreichend großes Spiel der Verbindungselemente von Kettenglied zu Kettenglied (Wirbel) erreicht werden.

Die Abstandhalter 5 können, wie bereits erwähnt, an ihren freien Enden mit Rollen oder Gleitschuhen zur leichteren Bewegung versehen sein. Sie sind bevorzugt zumindest in "Längsrichtung" elastisch, um über Unebenheiten durch elastische Deformation leichter hinweg zu kommen.

Die Sprühkette 18 kann mit zumindest einem Schlauch für das Desinfektionsmittel versehen sein, wobei zumindest an einer Stelle ein Auslass vorgesehen ist. Bevorzugt sind es mehrere gezielt zu öffnende Auslässe, die in verschiedene Umfangsrichtungen weisen, um Fluid in verschiedene Richtungen sprühen zu können. Das Vorsehen einer Kamera mit Datenübertragung nach Außen und gegebenenfalls einer Quelle für Licht im sichtbaren Wellenbereich an dieser Stelle / diesen Stellen ist zur Steuerung der Fluidabgabe und zur Kontrolle des Erfolges des Reinigungsvorganges vorteilhaft.

Es soll noch darauf hingewiesen werden, dass in der Beschreibung und den Ansprüchen Angaben wie "unterer Bereich" eines Gehänges, Reaktors, Filters, Bauwerks, oder einer Vorrichtung oder, ganz allgemein, eines Gegenstandes, die untere Hälfte und insbesondere das untere Viertel der Gesamthöhe bedeutet, "unterster Bereich" das unterste Viertel und insbesondere einen noch kleineren Teil; während "mittlerer Bereich" das mittlere Drittel der Gesamthöhe (Breite - Länge) meint. All diese Angaben haben ihre landläufige Bedeutung, angewandt auf die bestimmungsgemäße Position des betrachteten Gegenstandes.

In der Beschreibung und den Ansprüchen werden die Begriffe "vorne", "hinten", "oben", "unten" und so weiter in der landläufigen Form und unter Bezugnahme auf den Gegenstand in seiner üblichen Gebrauchslage, gebraucht. Das heißt, dass bei einer Waffe die Mündung des Laufes "vorne" ist, dass der Verschluss bzw. Schlitten durch die Explosionsgase nach "hinten" bewegt wird, etc.. Bewegungsrichtung bezieht sich, bei der Sprühkette auf die Bewegung beim Reinigungsvorgang.

In der Beschreibung und den Ansprüchen bedeutet "im Wesentlichen" eine Abweichung von bis zu 10 % des angegebenen Wertes, wenn es physikalisch möglich ist, sowohl nach unten als auch nach oben, ansonsten nur in die sinnvolle Richtung, bei Gradangaben (Winkel und Temperatur) sind damit ± 10° gemeint.

Alle Mengenangaben und Anteilsangaben, insbesondere solche zur Abgrenzung der Erfindung, soweit sie nicht die konkreten Beispiele betreffen, sind mit ± 10 % Toleranz zu verstehen, somit beispielsweise: 11 % bedeutet: von 9,9 % bis 12,1 %. Bei Bezeichnungen wie bei: "ein Lösungsmittel" ist das Wort "ein" nicht als Zahlwort, sondern als unbestimmter Artikel anzusehen, wenn nicht aus dem Zusammenhang etwas anderes hervorgeht.

Der Begriff: "Kombination" bzw. "Kombinationen" steht, soferne nichts anderes angegeben, für alle Arten von Kombinationen, ausgehend von zwei der betreffenden Bestandteile bis zu einer Vielzahl derartiger Bestandteile, der Begriff: "enthaltend" steht auch für "bestehend aus".

### Bezugszeichenliste:

- 1: Betriebstrommel
- 2: Versorgungsleitung
- 3: Rohr ( Fluidleiter )
- 4: Strahlenquelle
- 5: Abstandhalter
- 6: Sprühkopf
- 7: Desinfektionsmittelsprühnebel
- 8: Kupplung
- 9: Versorgungsleitungen aus der Betriebstrommel
- 10: Verbindungsadapter
- 11: Ab- bzw. Aufrolleinheit für den Verbindungsschlauch für Gas
- 12: Ab- bzw. Aufrolleinheit für den Verbindungsschlauch für Desinfektionsmittel
- 13: Ab- bzw. Aufrolleinheit für den Verbindungsschlauch für das Kabel für die Stromversorgung
- 14: Ab- bzw. Aufrolleinheit für den Verbindungsschlauch für das Kabel für die Kamera
- 15: Steuerungseinheit
- 16: Auslassrolle
- 17: Steuerleitungen
- 18: Sprühkette insgesamt
- 19: Verbindungsadapter
- 20: Biofilm

## Patentansprüche

1. Vorrichtung, so ausgebildet, dass sie zur Desinfektion von Innenwänden von Rohrleitungen (3) unter Verwendung von Desinfektionsmitteln und UV Licht geeignet ist, wobei sie zumindest einen Sprühkopf (6) für das Desinfektionsmittel und eine Quelle (4) für UV Licht aufweist, **dadurch gekennzeichnet, dass** der zumindest einen Sprühkopf (6) und die eine Quelle (4) auf einer Sprühkette (18) angeordnet sind, und wobei Abstandhalter (5) vorgesehen sind, die so ausgebildet sind, dass die Sprühkette (18) im Abstand von seitlichen Wandbereichen der Rohrleitungsinnenwand gehalten wird.

2. Vorrichtung nach Anspruch 1, **dadurch gekennzeichnet, dass** die Abstandhalter (5) zwei Endbereiche aufweisen und mit dem einem an der Sprühkette (18) angreifen und mit dem anderen, freien, Endbereich mit Gleitschuhen oder Rollen versehen sind.

3. Vorrichtung nach Anspruch 1 oder 2, **dadurch gekennzeichnet, dass** die Abstandhalter (5) in ihrer Länge einstellbar sind.

4. Vorrichtung nach einem der Ansprüche 1 bis 3, **dadurch gekennzeichnet, dass** zumindest drei Abstandhalter (5) vorgesehen sind, die in unterschiedliche Umfangsrichtung gerichtet sind.

5. Vorrichtung nach einem der Ansprüche 1 bis 4, **dadurch gekennzeichnet, dass** das Desinfektionsmittel aus der Gruppe, bestehend aus: halogen- und/oder sauerstoff- und/ oder stickstoffhältigen Chemikalien und/oder Alkohole ausgewählt ist.

6. Vorrichtung nach einem der voranstehende Ansprüche, **dadurch gekennzeichnet, dass** als Quelle (4) UVC bzw. UV LED Lampen verwendet werden.

7. Vorrichtung nach Anspruch 6, **dadurch gekennzeichnet, dass** Licht im Wellenlängenbereich zwischen 200 und 300 Nanometern (nm) verwendet wird.

8. Verwendung einer Vorrichtung nach einem der vorangegangenen Ansprüche zur Desinfektion von Rohrleitungen.

## Claims

1. A device designed to be suitable for disinfecting inner walls of pipelines (3) using disinfectants and UV light, wherein said device has at least one spray head (6) for the disinfectant and one source (4) for UV light, **characterized in that** the at least one spray head (6) and the one source (4) are arranged on a spray chain (18), and wherein spacers (5) are provided which are formed such that the spray chain (18) is retained spaced apart from lateral wall regions of the pipeline inner wall.

2. The device according to Claim 1, **characterized in that** the spacers (5) have two end regions and engage with the spray chain (18) with the one end region and are provided with sliding blocks or rollers on the other, free end region.

3. The device according to Claim 1 or 2, **characterized in that** the spacers (5) are adjustable in length.

4. The device according to any one of Claims 1 to 3, **characterized in that** at least three spacers (5) are provided which are oriented in different circumferential directions.

5. The device according to any one of Claims 1 to 4, **characterized in that** the disinfectant is selected from the group consisting of: chemicals which contain a halogen and/or oxygen and/or nitrogen, and/or alcohols.

6. The device according to any one of the preceding claims, **characterized in that** UVC or UV LED lamps are used as the source (4).

7. The device according to Claim 6, **characterized in that** light in the wavelength range of between 200 and 300 nanometers (nm) is used.

8. Use of a device according to any one of the preceding claims for disinfecting pipelines.

## Revendications

1. Dispositif, conçu de manière à se prêter à la désinfection de parois intérieures de tuyaux (3) au moyen de produits désinfectants et de lumière UV, présentant au moins une tête de pulvérisation (6) pour le produit désinfectant et une source (4) pour la lumière UV, **caractérisé en ce que** l'au moins une tête de pulvérisation (6) et la source (4) sont disposées sur une chaîne de pulvérisation (18), et dans lequel des écarteurs (5) conçus de telle manière que la chaîne de pulvérisation (18) est maintenue à distance de zones de parois latérales de la paroi intérieure de tuyau sont prévus.

2. Dispositif selon la revendication 1, **caractérisé en ce que** les écarteurs (5) présentent deux zones d'extrémité et s'appliquent avec l'une d'elles sur la chaîne de pulvérisation (18) et sont pourvus, avec l'autre zone d'extrémité libre, de patins ou de roulettes.

3. Dispositif selon la revendication 1 ou 2, **caractérisé en ce que** les écarteurs (5) sont réglables en longueur.

4. Dispositif selon l'une des revendications 1 à 3, **caractérisé en ce qu'**au moins trois écarteurs (5), orientés dans différentes directions circonférentielles, sont prévus.

5. Dispositif selon l'une des revendications 1 à 4, **caractérisé en ce que** le produit désinfectant est sélectionné dans le groupe constitué de : produits chimiques halogénés et/ou oxygénés et/ou azotés et/ou alcools.

6. Dispositif selon l'une quelconque des revendications précédentes, **caractérisé en ce que** des lampes à DEL UV-C ou UV sont utilisées comme source (4).

7. Dispositif selon la revendication 6, **caractérisé en ce que** de la lumière dans la plage de longueurs d'onde comprise entre 200 et 300 nanomètres (nm) est utilisée.

8. Utilisation d'un dispositif selon l'une quelconque des revendications précédentes pour la désinfection de tuyaux.
